# EUROPÄISCHE PATENTANMELDUNG

(11) **EP 1 035 210 A2**
(43) Veröffentlichungstag der Anmeldung: **13.09.2000**
(21) Anmeldenummer: 00104383.5
(22) Anmeldetag: 02.03.2000
(51) Int. Cl.: C12N 15/867, C12N 5/10, A61K 48/00

(54) **Von SIVagm abgeleitete lentivirale Vektoren, Verfahren zu ihrer Herstellung und ihre Verwendung zur Genübertragung in Säugerzellen**

(30) Priorität: 05.03.1999 DE 19909769
(71) Anmelder: Bundesrepublik Deutschland, letztvertreten durch den Präsidenten des Paul-Ehrlich-Instituts Prof. Dr. R. Kurth, 63225 Langen (DE)
(72) Erfinder: Cichutek, Klaus, Prof. Dr., 60598 Frankfurt a. Main (DE)
(74) Vertreter: Vossius, Volker, Dr.

(57) **Zusammenfassung**

Beschrieben ist die Herstellung und Verwendung neuer lentiviraler, von SIVagm abgeleiteter Vektoren für den Gentransfer, insbesondere ein Herstellungsverfahren der lentiviralen Vektoren, die Kapsidpartikel des Simianen Immundefizienzvirus SIVagm und Hüllproteine des SIVagm und anderer Retroviren wie dem humanen Immunschwächeviren (HIV), anderer Affen-Immunschwächeviren (SIV), anderer Retroviren wie dem Murinen Leukämievirus (MLV) oder dem "Gibbon Ape Leukemia Virus" (GaLV) oder dem Porcinen endogenen oder exogenen Retrovirus (PERV), des "Vesicular Stomatitis Virus" (VSV-G)enthalten. Die Vektoren und entsprechende Verpackungszellen können für die Verpackung und den Transfer von Genen eingesetzt werden, die von anderen lentiviralen, retroviralen und anderen Vektoren nicht verpackt werden oder deren Gentransfer mit anderen Vektorpartikeln nicht effizient verläuft. Diese Vektoren können für die Genübertragung in ausgewählte Zelltypen, speziell in humane proliferationsaktive und ruhende Zellen verwendet werden.

## Beschreibung

Gegenstand der Erfindung sind von SIVagm (AGM; Afrikanische Grüne Meerkatze; Cercopithecus bzw. Chlorocebus) abgeleitete retrovirale Vektoren (sogenannte lentivirale Vektoren), Verfahren zu ihrer Herstellung und ihrer Verwendung zur Genübertragung in Säugerzellen.

Der Ausdruck "lentivirale Vektoren" oder "SIVagm-Vektoren" bezeichnet infektiöse, aber vermehrungsunfähige Retroviren, die Gene in Form von retroviralen Expressionsvektoren (auch Expressionskonstrukte oder verpackbare Konstrukte genannt) in Zellen einschleusen können. Als Lentiviren wird eine Gruppe der Retroviridae bezeichnet, die nach Infektion des Menschen, anderer Primaten und Säuger (z.B. Schafe, Katzen) nach langer Inkubationszeit zur Krankheit führt. Der Gentransfer mit Retroviren bzw. Lentiviren wird auch als Transduktion bezeichnet. Die Genübertragung führt zur Integration des Expressionsvektors in das Genom der Zelle. Expressionsvektoren beinhalten ein Verpackungssignal psi, welches zur Inkorporation der RNA des Expressionsvektors in die Vektorpartikel und zur Genübertragung führt. Als "psi" wird also das Verpackungssignal der Retroviren bezeichnet, das die effiziente Verpackung der Boten-RNA des Expressionsvektors steuert. Der Expressionsvektor muß zudem von lentiviralen LTR-Sequenzen ("Long Terminal Repeats") flankiert sein, damit die korrekte Umschreibung der RNA des Expressionsvektors in DNA und die nachfolgende Integration des Expressionvektorgens in die chromosomale DNA der Zelle erfolgt. Der lentivirale Gentransfer ist vorteilhaft, weil (i) in der Regel eine Kopie des gewünschten Gens in Zellen überführt wird, (ii) das Gen im allgemeinen ohne Mutationen oder Umlagerungen transferiert wird, (iii) ein stabiler chromosomaler Einbau erfolgt und (iv) Gene auch in nicht-proliferierende Zellen übertragen werden können.

Es ist bekannt, lentivirale Vektoren auf der Basis des Humanen Immundefizienzvirus des Typs 1 (HIV-1), des Typs 2 (HIV-2) und des Simianen Immundefizienzvirus des Rhesusaffen (Macaca mulatta) zu benutzen, um bestimmte Gene in Säugerzellen, speziell auch humane Zellen, zu überführen. Ein besonderer Vorteil der lentiviralen Vektoren besteht in ihrer Fähigkeit, auch ruhende, bzw. sich nicht teilende Zellen zu transduzieren. Diese Vektoren sind vermehrungsunfähig und durchlaufen lediglich eine Replikationsrunde. Zur Herstellung derartiger Vektoren benötigt man drei Komponenten. Innerhalb einer Verpackungszelle benötigt man ein psi-negatives *gag/pol*-Gen des ursprünglichen Lentivirus, ein psi-negatives env-Gen, das von Lentiviren oder anderen Viren abgeleitet sein kann, und ein psi-positives und damit verpackbares Expressionskonstrukt, das im allgemeinen auch von einem Lentivirus abgeleitet ist. Der Expressionsvektor ermöglicht die Verpackung in den retroviralen Vektor und den Transfer durch das Retrovirus, so daß eine kodierende und übersetzungsfähige Region des gewünschten Genprodukts in die Zielzelle überführt wird. Nach der Überführung der Plasmide, welche das *gag/pol*-Gen, das env-Gen und das Expressionsvektorgen enthalten, durch Transfektion der drei entsprechenden DNAs in eine geeignete Säugerzelle entsteht eine Verpackungszelle, welche retrovirale Vektorpartikel in den Zellüberstand abgibt, die das Expressionskonstrukt enthalten und nur dieses Konstrukt, nicht jedoch die psi-negativen *gag/pol*- und env-Gene, so daß diese nicht in Zielzellen überführt werden.

Der Tropismus der lentiviralen Vektoren, d. h. die Auswahl der Säugerzellen, in welche diese das Expressionkonstrukt überführen können, wird durch das env-Gen in der benutzten Verpackungszelle und damit durch die env-Genprodukte in den Vektorpartikeln bestimmt. Das env-Gen der Retroviren, von denen z.B. das murine Leukämievirus (MLV), verschiedene Lentiviren wie HIV, SIV oder FIV ("Felines Immundefizienzvirus), aber auch EIAV ("Equine Infectious Anemia Virus") oder CIAV ("Caprine Infectious Anemia Virus") zur Bildung lentiviraler Vektorpartikel eingesetzt werden, wird in Hüllproteine, das Transmembranprotein (TM) und das Oberflächenhüllprotein (SU), übersetzt, welche die äußere Hülle des lentiviralen Vektors bilden. Die env-Genprodukte des amphotropen MLV, des GaLV ("Gibbon Ape Leukemia Virus") und das G-Protein des VSV ("Vesicular Stomatitis Virus", Burns et al., Proc. Natl. Acad. Sci. USA 90 (1993), 8033-8037) werden bisher vor allem für den Gentransfer benutzt. Sie erlauben den Gentransfer in eine große Anzahl unterschiedlicher Säugerzellen, auch in humane Zellen. Speziell für den selektiven Gentransfer in humane Zellen eines bestimmten Zelltyps, z.B. T-Zellen oder hämatopoetische Stammzellen, sind die env-Genprodukte des ecotropen, des amphotropen MLV oder des Milznekrosevirus (SNV; "Spleen Necrosis Virus") geeignet, wenn sie durch Einbau von Domänen einzelkettiger Antikörper (scFv; "single chain Fv") oder anderer Liganden für Zelloberflächenproteine wie z.B. der Zytokine oder Wachstumsfaktoren modifiziert wurden.

Zur Verbesserung der Übertragung unterschiedlicher Gene wurden retrovirale Vektoren vorgeschlagen, bei denen statt des *gag/pol*-Gens der Onko-Retroviren wie MLV, das *gag/pol*-Gen unterschiedlicher Lentiviren wie HIV-1, HIV-2, SIVmac, FIV oder EIAV eingesetzt wurde. Der Erfindung liegt daher die Aufgabe zugrunde, verbesserte lentivirale Vektoren (retrovirale Viruspartikel) bereitzustellen.

Die Aufgabe wird durch die Patentansprüche gelöst.

Ein besonderer Vorteil der von SIVagm abgeleiteten letiviralen Vektoren besteht auch darin, daß (i) bei Verwendung der SIVagm-Hüllproteine keine oder eine geringe Menge von Antikörpern, insbesondere neutralisierende Antikörper, gegen den Vektor gebildet werden. Damit sind Mehrfach-Anwendungen möglich, die bei anderen lentiviralen Vektoren nicht möglich sind. Zum anderen ist auch die Anwendung in Gegenwart von anti-HIV-Antikörpern möglich, welche den Gentransfer mit SIVagm-Vektoren mit homologer SIVagm-Hülle nicht oder nur wenig behindern. Eine weitere Besonderheit der SIVagm-Vektoren besteht darin, daß mit ihrer Hilfe Gene verpackt und in Säugerzellen überführt werden können, welche die Bildung entsprechender von HIV- oder anderer Lentiviren abgeleiteter Vektorpartikel behindern würden. Beispielsweise können gegen die HIV-1Reverse Transkriptase oder -Integrase gerichtete Antikörpergene (anti-RT-scFv- und anti-Int-scFv-Gene) mit Hilfe der SIVagm-Vektoren verpackt und zum Gentransfer verwendet werden, während HIV-Vektoren nicht oder nur sehr wenig effizient für solche Gentransfers benutzt werden können.

In einer erfindungsgemäßen Ausführungsform wird eine beliebige Zelle mit einem psi-negativen Expressionsgen für gag- und pol-Gene des SIVagm transfiziert. Ferner kann die Zelle mit einem Expressionskonstrukt, umfassend ein psi-Verpackungssignal und die in eine Zielzelle zu überführende genetische Information, transfiziert werden. Das Expressionskonstrukt kann von SIVagm, SIVmac oder HIV abgeleitet sein, muß jedoch die Verpackung und Umschreibung in Begleitung mit der Verwendung der enzymatischen Genprodukte des pol-Gens und der Kapsid-Genprodukte des gag-Gens des SIVagm erlauben. Die Zelle wird dann mit einem weiteren Expressionsgen transfiziert, das die genetische Information für fremde oder eigene Hüllproteine enthält. Die so hergestellte Zellinie produziert lentivirale, von SIVagm abgeleitete Vektoren, welche die zu überführende genetische Information enthalten.

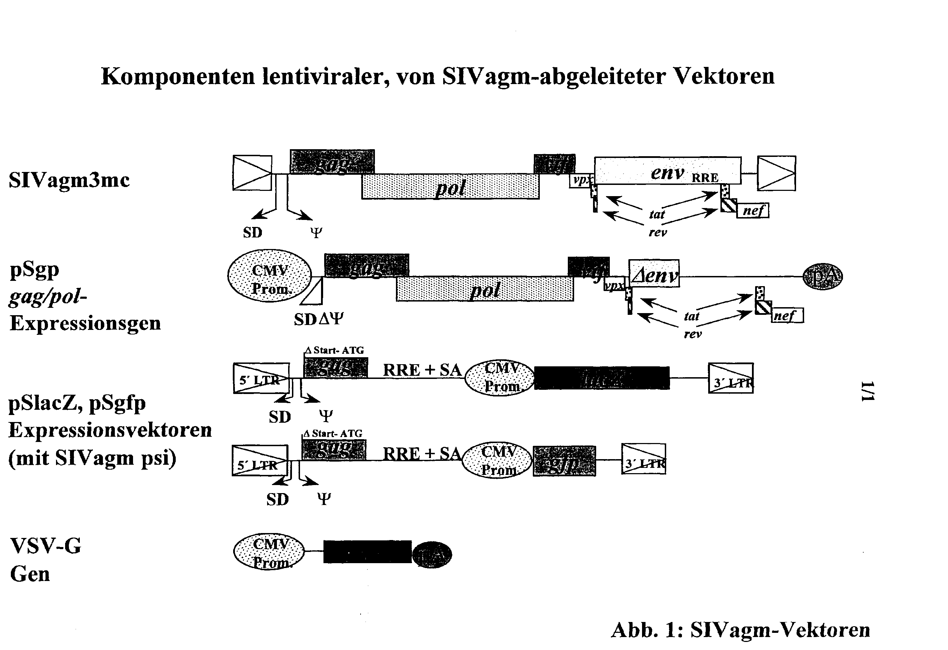

In einer bevorzugten Ausführungsform wird die humane Zellinie 293T mit dem *gag/pol*-Gen des SIVagm, dem env-Gen des SIVagm und dem verpackbaren Expressionsvektor pSgfp (siehe Abb. 1) gleichzeitig transfiziert. Alternativ werden Plasmide eingesetzt, die das G-Protein des VSV, die modifizierten env-Genprodukte des SNV, die env-Genprodukte des MLV, die Hybrid-env-Genprodukte des GaLV mit lentiviralen C-Termini kodieren. Dabei entsteht eine Verpackungszellinie, die die erfindungsgemäßen lentiviralen, von SIVagm abgeleiteten Vektoren in den Zellkulturüberstand abgibt. Diese Vektoren enthalten Kapsidpartikel, die aufgrund der Expression des *gag/pol*-Gens des SIVagm in der Zelle entstehen, und verpackbare, lentivirale Expressionsvektor-RNA mit den zu transferierenden Genen. Die Vektorpartikel enthalten auch die Hüllproteine des SIVagm, welche durch die intrazelluläre Expression des env-Gens des SIVagm in die Vektorpartikel aufgenommen werden. Alternativ bilden die anderen angegebenen env-Genprodukte die Virushülle. Im einzelnen wurde nachgewiesen, daß dies zur Herstellung lentiviraler Vektoren für den Gentransfer in proliferationsaktive oder ruhende Säugerzellen führte. Die Transduktion durch SIVagm-Vektorpartikel mit SIVagm-Env-Hülle wurde in Gegenwart von Antikörpern gegen HIV nicht gehemmt. Die angegebenen Oberflächenhüllproteine des SIVagm und der anderen Viren wurden auf der Oberfläche der hergestellten Gentransfervektoren nachgewiesen.

Die vorliegende Erfindung eröffnet die folgenden Möglichkeiten:
- Gene in proliferationsaktive Säugerzellen zu überführen,
- Gene in ruhende Säugerzellen zu überführen,
- Gene in proliferationsaktive und ruhende CD4-positive Säugerzellen zu überführen,
- Gene in proliferationsaktive und ruhende hämatopoetische Stamm- und Vorläuferzellen zu überführen,
- Gene in proliferationsaktive und ruhende neuronale Zellen zu überführen,
- Gene in proliferationsaktive und ruhende andere humane Stammzellen zu überführen,
- Gene in proliferationsaktive und ruhende humane CD4-positive Zellen zu überführen,
- in Gegenwart von anti-HIV-Antikörpern Gene in proliferationsaktive und ruhende humane CD4-positive Zellen zu überführen,
- insbesondere Gentherapiestrategien zur Behandlung oder Prophylaxe der HIV-Infektion des Menschen zu entwickeln, in dem HIV-hemmende Gene, z.B. Antisensegene, RNA-Ködergene oder transdominant-negative mutante Gene des HIV oder anderer Lentiviren in bestimmte Zellen überführt werden,
- im Rahmen von Gentherapiestrategien zur Behandlung oder Prophylaxe der HIV-Infektion des Menschen Vektoren zu entwickeln, welche HIV-hemmende oder andere Gene, die mit Hilfe von HIV abgeleiteten Vektoren nicht verpackt und überführt werden können, in bestimmte Zellen überführen,
- insbesondere Gentherapiestrategien zur Einführung von Genen in bestimmte Zellen zu entwickeln, zur Behandlung oder Prophylaxe von Erbkrankheiten, wie z.B. der ADA-Defizienz oder anderen genetisch behandelbaren Krankheiten, bei denen die Einführung in bestimmte Zellen vorteilhaft ist, wie z.B. T-Zellymphome, Leukämien oder andere Tumorerkrankungen und
- den Eintritt von Lentiviren in Säugerzellen und die Bildung lentiviraler Virionen im Detail zu untersuchen.

Die Abbildung dient der Erläuterung der Erfindung.

Abbildung 1 zeigt schematisch die Struktur von zwei auf SIVagm basierenden Expressionsvektoren, des auf SIVagm basierenden *gag/Pol*- und des env-Gens.

Der hier verwendete Begriff lentiviraler Vektor bedeutet replikationsdefizientes, von SIVagm abgeleitetes, retrovirales Viruspartikel mit homologer oder von anderen Viren abstammender Hülle, das anstelle der retroviralen mRNA eine fremde eingeführte RNA eines Gens übertragen kann, z.B. eines therapeutischen Gens oder dessen Fragment oder eines Reportergens.

Der hier verwendete Begriff "therapeutisches Gen" bedeudet eine Nukleinsäuresequenz, die in die Zielzelle mittels des retroviralen Vektors eingeführt werden soll und umfaßt komplette Gene, deren Fragmente, Antisense-Nukleinsäuren und dergleichen. Der hier verwendete Begriff SIV bedeutet Viren der Familie des Affen-Immunschwächevirus, z.B. laut *FIELDS, Virology "Cercopithecus"aethiops* (SIVagm), neuerdings umbenannt in *Chlorocebus, Macaca mulatta* (SIVmac), *Pan troglodydytes* (SIVcpz), *Cerecopithecus mitis* (SIVsyk), *Papio sphinx* (SIVmnd), *Cercocebus atys* (SIVsm) oder *Maccaca nemestrina* (SIVmne).

Die nachfolgenden Beispiele erläutern die Erfindung und sind nicht einschränkend zu verstehen.

### 1. Beispiel 1: Herstellung von SIVagm-Vektoren mit homologer Hülle

Alle hier verwendeten Gene oder nicht-codierende Regionen des SIVagm entstammen dem Molekularklon SlVagm3mc (SWISS-PROT Accession number M30931). Zur Herstellung von Vektorpartikeln wurden 293T-Zellen verwendet. Diese wurden in DMEM mit 10 % FKS expandiert und 24 h vor Transfektionsbeginn in Sechslochplatten (Fa. Nunc, Wiesbaden) mit 5 x 10⁵ Zellen pro Bohrung ausgesät. Als Transfektionsreagenz wurde Lipofectamin PLUS (Fa. Gibco, Eggenstein) nach den Angaben des Herstellers verwendet. Hierzu wurden je 1µg Plasmid DNA der von SIVagm3mc abgeleiteten Genkonstrukte, welche für *gag/pol, env* und das verpackbare Expressionskonstrukt codieren, transfiziert. Nach vierstündiger Inkubation der 293T-Zellen mit den oben beschriebenen DNA-Komplexen wurden die Zellen gewaschen und mit frischem Medium versorgt. Zwei Tage nach der Transfektion wurden die Zellkulturüberstände geerntet und durch Filtration durch Spritzenfilter (Porengröße 0,45 µm) von kontaminierenden Verpackungszellen befreit.

### 2. Beispiel 2: Herstellung von SIVagm- Vektoren mit der unmodifizierten Hülle anderer Retroviren

Zur Herstellung von sogenannten Pseudotypvektoren wurde methodisch exakt so verfahren wie in Beispiel 1 beschrieben. Jedoch wurden anstelle der homologen Hüllen des SIVagm, heterologe Hüllproteingene *(env)* anderer Viren verwendet. Diese können vom amphotrophen MLV (Konstrukt pHIT456, Soneoka et al.), vom 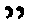 Vesicular Stomatitis Virus" (pMD-G, Naldini et al.) oder anderen Viren stammen. Die so generierten Vektorpartikel bestehen aus Viruskernen und einem Expressionsvektor, welche von SIVagm3mc abgeleitet sind und den jeweilig verwendeten heterologen Hüllproteinen.

### 3. Beispiel 3: Herstellung von SIVagm-Vektoren mit modifizierter Hülle

Zur Herstellung von SIVagm-Vektoren mit modifizierter Hülle wird ebenfalls wie bereits oben beschrieben verfahren. Jedoch werden hier modifizierte *env*-Gene verwendet, welche z. B. von SNV *env* abgeleitet sein können. Diese beinhalten codierende Regionen von Antikörpern oder Rezeptorliganden.

## Patentansprüche

1. SIVagm-Vektoren umfassend Viruskerne und Virushüllen, dadurch gekennzeichnet, daß die Viruskerne vom Simianen Immundefizienzvirus (SIVagm) der Afrikanischen Grünen Meerkatze (Chlorocebus, vorher Cercopithecus aethiops) und die Virushüllen von SIVagm oder anderen Viren stammen.

2. SIVagm-Vektoren nach Anspruch 1, dadurch gekennzeichnet, daß die Virushüllen vom menschlichen Immunschwächevirus 1 oder 2 (HIV-1, bzw. HIV-2) oder Affen-Immunschwächevirus Cerecopithecus aethiops (SIVagm) oder Macaca mulatta (SIVmac) oder Pan troglodydytes (SIVcpz) oder Cerecopithecus mitis (SIVsyk) oder Papio sphinx (SIVmnd) oder Cercocebus atys (SIVsm) oder Maccaca nemestrina (SIVmne) stammen.

3. SIVagm- Vektoren nach Anspruch 1 oder 2, dadurch gekennzeichnet, daß die Virushüllen des murinen ectotropen oder amphotropen Leukämievirus (MLV), des avianen Milznekrosevirus (SNV), des GaLV ("Gibbon Ape Leukemia Virus"), des Porcinen endogenen oder Porcinen exogenen Retrovirus (PERV) verwendet werden.

4. SIVagm- Vektoren nach einem der Ansprüche 1 bis 3, dadurch gekennzeichnet, daß die Virushüllen durch einzelkettige Antikörper (scFv) oder andere Liganden (z.B. Zytokinen) von Zelloberflächenmolekülen modifziert wurden.

5. SIVagm- Vektoren nach einem der Ansprüche 1 bis 4, dadurch gekennzeichnet, daß die Virushüllen von unterschiedlichen Viren stammen.

6. SIVagm-Vektoren nach einem der Ansprüche 1 bis 5, geeignet zur Verpackung und Überführung von Genen in Säugerzellen, die von anderen lentiviralen oder retroviralen Vektoren nicht verpackt oder überführt werden können, z.B. im Rahmen von Gentherapiestrategien zur Behandlung oder Prophylaxe der HIV-Infektion des Menschen.

7. Verfahren zur Herstellung von Verpackungszellen, die von SIVagm abgeleitete Vektoren nach einem der Ansprüche 1 bis 6 bilden, umfassend das Transfizieren einer Empfängerzelle oder einer Verpackungszelle (env- und Expressionskonstrukt-positiv), die Virushüllen produziert oder nicht produziert (env-negativ), mit einem *gag/pol*-Gen, das die genetische Information für SIVagm-Kapsidpartikel enthält.

8. Verfahren zur Herstellung von Verpackungszellen, die lentivirale Vektoren nach einem der Ansprüche 1 bis 6 bilden, umfassend das Transfizieren einer Zelle mit Expressionsgenen für gag und pol, und/oder einem Expressionskonstrukt, umfassend ein Verpackungssignal und die zu überführende genetische Information, und einem Expressionsgen, das die genetische Information für Hüllproteine enthält.

9. Verfahren nach einem der Ansprüche 7 bis 8, wobei als Expressionsvektorgene pSgfp oder pSlacZ (siehe Abb. 1) verwendet werden.

10. Verpackungszellen, erhältlich nach dem Verfahren nach einem der Ansprüche 7 bis 8.

11. Verwendung der lentiviralen Vektoren nach einem der Ansprüche 1 bis 6 oder der Verpackungszellen nach Anspruch 10 als Arzneimittel.

12. Verwendung der lentiviralen Vektoren nach einem der Ansprüche 1 bis 6 oder der Verpackungszellen nach Anspruch 10 zur Herstellung eines Arzneimittels zur Überführung von Genen in Zellen.

13. Verwendung nach Anspruch 12, wobei die Zellen humane Zellen sind.

14. Verwendung der lentiviralen Vektoren nach einem der Ansprüche 1 bis 6 und entsprechender Verpackungszellen nach Anspruch 10 zur Herstellung eines Arzneimittels zur gentechnischen Modifizierung von Zellen oder zur Bereitstellung eines Wirkstoffs im Rahmen der Gentherapie.
